(19) Europäisches Patentamt
European Patent Office
Office européen des brevets

(11) Veröffentlichungsnummer : **0 393 428 B1**

(12) # EUROPÄISCHE PATENTSCHRIFT

(45) Veröffentlichungstag der Patentschrift : **27.10.93 Patentblatt 93/43**

(51) Int. Cl.[5] : **C07D 303/44,** C07D 301/03, C07C 69/734, A01N 43/04

(21) Anmeldenummer : **90106525.0**

(22) Anmeldetag : **05.04.90**

(54) **Oxiran-phenylester und diese enthaltende Fungizide.**

Die Akte enthält technische Angaben, die nach dem Eingang der Anmeldung eingereicht wurden und die nicht in dieser Patentschrift enthalten sind.

(30) Priorität : **11.04.89 DE 3911724**
**23.05.89 DE 3916719**

(43) Veröffentlichungstag der Anmeldung : **24.10.90 Patentblatt 90/43**

(45) Bekanntmachung des Hinweises auf die Patenterteilung : **27.10.93 Patentblatt 93/43**

(84) Benannte Vertragsstaaten : **AT BE CH DE DK ES FR GB GR IT LI NL SE**

(56) Entgegenhaltungen :
**EP-A- 0 228 045**
**EP-A- 0 229 974**

(73) Patentinhaber : **BASF Aktiengesellschaft**
**Carl-Bosch-Strasse 38**
**D-67063 Ludwigshafen (DE)**

(72) Erfinder : **Seele, Rainer, Dr.**
**Leiblstrasse 3**
**D-6701 Fussgoenheim (DE)**
Erfinder : **Schuetz, Franz, Dr.**
**Budapester Strasse 45**
**D-6700 Ludwigshafen (DE)**
Erfinder : **Wingert, Horst, Dr.**
**D 3, 4**
**D-6800 Mannheim 1 (DE)**
Erfinder : **Sauter, Hubert, Dr.**
**Neckarpromenade 20**
**D-6800 Mannheim 1 (DE)**
Erfinder : **Ammermann, Eberhard, Dr.**
**Sachsenstrasse 3**
**D-6700 Ludwigshafen (DE)**
Erfinder : **Lorenz, Gisela, Dr.**
**Erlenweg 13**
**D-6730 Neustadt (DE)**

**EP 0 393 428 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Ester, die einen Oxiranrest und einen Phenylrest enthalten, diese enthaltende Fungizide und ihre Verwendung als Fungizide.

Es ist bekannt, N-Tridecyl-2,6-dimethylmorpholin (DE-1 164 152) α-Phenyl-β-methoxy-acrylsäuremethylesterderivate (DE-3 519 282.8) oder α-Ethylphenyl-β-methoxy-acrylsäuremethylesterderivate (EP-A-0 229 974) als Fungizide zu verwenden. Ihre Wirkung ist jedoch in manchen Fallen ungenügend.

Es wurde nun gefunden, daß neue Ester der Formel I

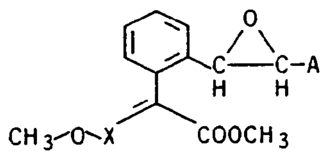

I,

in welcher

A Wasserstoff, $C_1$-$C_6$-Alkyl, $C_3$-$C_6$-Cyloalkyl, $C_5$-$C_6$-Cycloalkenyl, Naphthyl, Biphenyl, Pyridyl, Furyl, Thienyl, Oxazolyl, 1,3-Oxazolyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

X den Rest N oder CH bedeutet,

eine bessere fungizide Wirkung besitzen als bekannte Acrylester.

Die neuen Verbindungen der Formel I können bei der Herstellung als Gemische von Stereoisomeren (E/Z-Isomere, Diastereomere, Enantiomere) anfallen, die in üblicher Weise, z.B. durch Kristallisation oder Chromatographie, in die Einzelkomponenten getrennt werden können. Sowohl die einzelnen Isomeren als auch ihre Gemische könne als Fungizide verwendet werden und werden von der Erfindung erfaßt.

A bedeutet beispielsweise $C_1$-$C_6$-Alkyl, ($C_1$-$C_4$-Alkyl), Methyl, Ethyl, Propyl, Butyl oder $C_3$-$C_6$-Cycloalkyl, Cyclopropyl, Cyclohexyl oder $C_5$-$C_6$-Cycloalkenyl, Cyclohexenyl, Pyridyl, Furyl, Thienyl, Oxazolyl, 1,3-Oxazolyl. Als Substituenten werden beispielsweise genannt Halogen (F, Cl, Br, J), $C_1$-$C_4$-Alkyl (Methyl, Ethyl, Propyl, Butyl), $C_1$-$C_4$-Alkoxy (Methoxy, Ethoxy, Propoxy), Halogenalkyl (Trifluormethyl). A bedeutet beispielsweise Chlorphenyl, Fluorphenyl, Bromphenyl, Methoxyphenyl, Phenoxyphenyl, tert.-Butylphenyl.

Die Verbindungen der Formel I können z.B. hergestellt werden, indem man eine Verbindung der Formel III

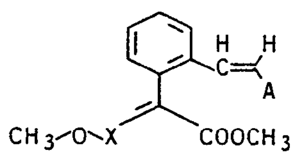

III,

in welcher A und X die oben angegebenen Bedeutungen haben, in die Epoxide überführt. Dazu oxidiert man die Olefine III mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat. Man arbeitet zwischen 10 und 100°C und katalysiert die Reaktion gegebenenfalls z.B. mit Jod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30°C sowie Alkylhydroperoxide, z.B. tert.-Butylhydroperoxid, unter Zusatz eines Katalysators z.B. Natriumwolframat, Perwolframsäure, Molybdänhexacarbonyl oder Vanadylacetylacetonat. Die genannten Oxidationsmittel lassen sich z.T. in situ erzeugen.

Die Verbindungen der Formel III können nach bekannten Verfahren hergestellt werden (vgl. EP 178826, EP 203606 und EP 253213).

Weiterhin erhält man die Verbindungen der allgemeinen Formel I, in denen X den Rest CH bedeutet, aus den substituierten α-Aryl-β-hydroxy-acrylsäure-methylesterderivaten der allgemeinen Formel VII, die im Gleichgewicht mit den Formylderivaten VIII vorliegen können, durch Umsetzung mit einem Alkylierungsmittel

(z.B. Dimethylsulfat oder Methyljodid) in Gegenwart einer Base (z.B. Kaliumcarbonat oder Natriumcarbonat) in einem Verdünnungsmittel (z.B. Aceton). In den folgenden Formelbildern bedeutet "L" eine Abgangsgruppe (z.B. Methylsulfat oder Jodid).

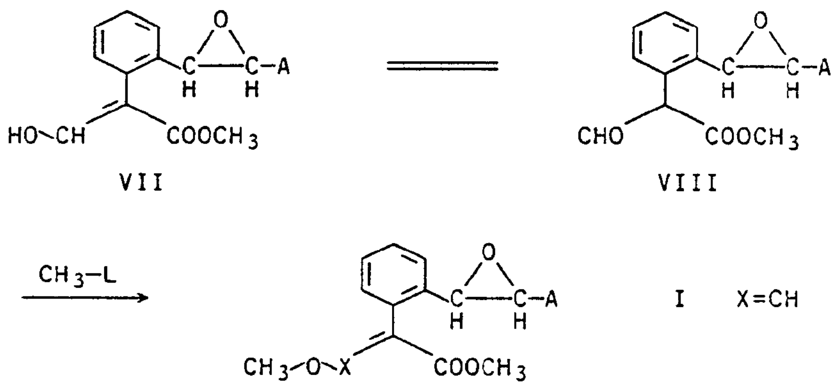


VII VIII

I X=CH

Die α-Aryl-β-hydroxyacrylsäuremethylester der allgemeinen Formel VII erhalt man aus den substituierten Phenylessigsäuremethylestern der allgemeinen Formel II durch Umsetzung mit Ameisensäuremethylester unter Verwendung einer Base (z.B. Natriumhydrid, Lithiumdiisopropylamid oder Natriummethanolat) in einem inerten Losungsmittel wie z.B. Diethylether oder Tetrahydrofuran (vgl. dazu Ann. Chem. 424 (1921) 214).

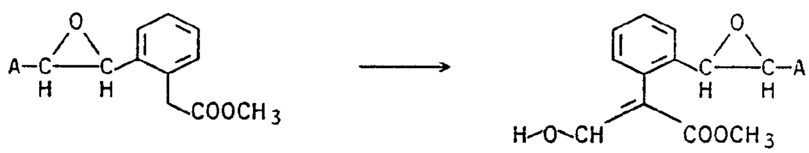

II VII

Die Verbindungen der Formel I, in denen A den Rest Wasserstoff bedeutet, können hergestellt werden, indem man eine Verbindung der Formel IV

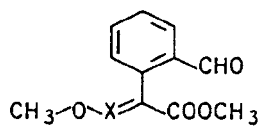

IV,

in welcher X den Rest CH bedeutet, mit einem Schwefelylid der Formel V

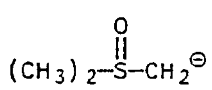

V,

umsetzt (vgl. Corey, Chaykovsky, J.A.C.S. 1962, 84, 3782).

Die Verbindungen der Formel IV können nach bekannten Methoden hergestellt werden (vgl. EP 278595).

Verbindungen der Formel I, in denen A den Rest Wasserstoff und X den Rest N bedeutet, können in entsprechender Weise hergestellt werden, indem man eine Verbindung der Formel IV, in welcher X den Rest N bedeutet, als Ausgangsprodukt verwendet.

Die Verbindung der Formel IV, in der X den Rest N bedeutet, kann wie folgt hergestellt werden:

2-Formylphenyl-glyoxylsäuremethylester-O-methyloxim

20 g (0,07 mol) 2-Brommethylphenyl-glyoxylsäuremethylester-O-methyloxim werden in 300 ml $CCl_4$ gelöst. Man gibt 32 g (0,237 mol) Methylmorpholin-N-oxid-monohydrat zu und erhitzt 9 Stunden unter Rückfluß. Der Feststoff wird abfiltriert. Die $CCl_4$-Phase wird mit Wasser, mit 2 N HCl und wieder mit Wasser gewaschen,

getrocknet und eingeengt. Der Rückstand wird aus Pentan umkristallisiert. Man erhält 9,0 g des Produkts (58 %) als farblose Kristalle. m.p. 100-105°C; IR ($cm^{-1}$): 1725, 1690, 1443, 1214, 1204, 1074, 1042, 1020, 951, 756.

Die Styroloxide der Formel I, in denen A den Rest Wasserstoff und X den Rest CH oder N bedeutet,

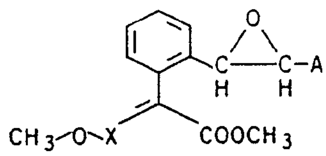

I,

sind als Fungizide geeignet und stellen zusätzlich Zwischenprodukte für die Synthese neuer Esterderivate dar.

Diese Styroloxide eignen sich besonders zur Herstellung von anderen fungiziden Wirkstoffen durch Umsetzung mit verschiedenen Nukleophilen, z.B. mit Phenol.

Die Verbindungen der Formel II können hergestellt werden, indem man eine Verbindung der Formel VI

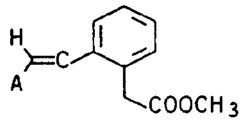

VI,

in welcher A die oben angegebene Bedeutung hat, in das entsprechende Epoxid überführt. Hierbei oxidiert man die Olefine VI mit Peroxycarbonsäuren, wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natrium acetat, Natriumcarbonat, Dinatriumhydrogenphosphat. Man arbeitet zwischen 10 und 100°C und katalysiert die Reaktion gegebenenfalls z.B. mit Jod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30°C sowie Alkylhydroperoxide z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Die Herstellung der als Ausgangsverbindungen benötigten substituierten Phenylessigsäuremethylester VI erfolgt durch Umsetzung von einem 2-Formylphenylessigsäuremethylester IX mit einem Methanphosphonsäureester der allgemeinen Formel X (A hat die oben angegebene Bedeutung, $R^1$=Methyl oder Ethyl). Die Durchführung der Reaktion erfolgt in an sich bekannter Weise (vgl. z.B. J. Am. Chem. Soc. 83 (1961) 1733). Die Ausgangsstoffe werden üblicherweise in einem stöchiometrischen Verhältnis eingesetzt. Ein Überschuß bis zu 10 % (Gew.%) an einem der beiden Reaktionsteilnehmer über die stöchiometrische Menge hinaus ist möglich. Die Umsetzung wird zweckmäßig in einem inerten Lösungs- oder Verdünnungsmittel (z.B. Diethylether, Tetrahydrofuran, Methyl-tert.-butylether, Dimethoxyethan, Toluol, Dimethylsulfoxid) in Gegenwart einer äquivalenten Menge einer Base (z.B. Natriumhydrid, Natriumamid, Kalium-tert.-butylat, Natriummethanolat, Butyllithium, Phenyllithium, Natrium-bis-trimethylsilylamid, Natriummethylsulfinylmethyl) durchgeführt. Die Umsetzungen verlaufen gewöhnlich in einem Temperaturbereich von -70°C bis 30°C. Da sie in einigen Fällen unter Wärmeentwicklung verlaufen, kann es von Vorteil sein, Kühlmöglichkeiten vorzusehen.

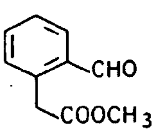

IX

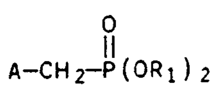

X

2-Formyl-phenylessigsäuremethylester IX erhält man durch Veresterung von 2-Formyl-phenylessigsäure XI mit Methanol unter Standardbedingungen. Die Darstellung von 2-Formyl-phenylessigsäure XI gelingt in einfacher Weise durch Ozonolyse des Trimethylsilylenolethers XIII von 2-Indanon XII (Tetrahedron Lett.25 (1984)

3659; Tetrahedron 43 (1987) 2075).

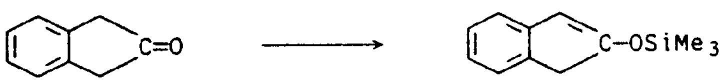

XII XIII

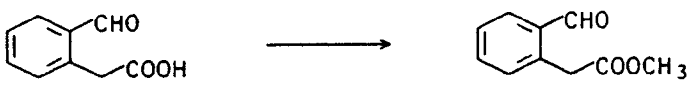

XI IX

Die substituierten Methanphosphonsäureester der allgemeinen Formel X (A hat die oben angegebene Bedeutung, $R^1$=Methyl oder Ethyl) erhält man durch Umsetzung der entsprechenden Methylhalogenverbindungen mit Phosphorigsäuretrimethylester oder Phosphorigsäuretriethylester $P(OR^1)_3$ (vgl. Methoden der organischen Chemie, Band 12/1, S, 433, Thieme, Stuttgart 1963).

Die folgenden Beispiele und Vorschriften erläutern die Herstellung der Wirkstoffe und ihrer Vorprodukte.

Vorschrift 1

2-(2′-Phenyl-ethen-1′-yl)-phenylessigsäuremethylester

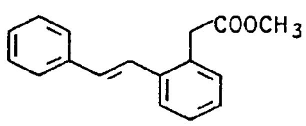

Zu einer Suspension von 24,0 g (0,062 mol) Benzyl-triphenylphosphoniumchlorid in 300 ml Diethylether werden bei Raumtemperatur (20°C) 7,0 g (0,057 mol) Kalium-tert.-butylat gegeben. Man rührt noch 30 Minuten bei Raumtemperatur, kühlt auf 10°C ab und gibt anschließend 10,0 g (0,056 mol) 2-Formyl-phenylessigsäure in 20 ml Diethylether zu. Die Reaktionsmischung wird noch 12 Stunden bei Raumtemperatur gerührt. Man hydrolysiert mit 300 ml Wasser, extrahiert mit Diethylether und entfernt das Lösungsmittel am Rotationsverdampfer. Der Rückstand wird in n-Hexan aufgenommen und das ausgefallene Triphenylphosphinoxid abgetrennt. Das Filtrat wird eingeengt und über Kieselgel (Cyclohexan/Essigester=9:1) chromatographiert. Man erhält 11,8 g (83 %) 2-(2′-Phenyl-ethen-1′-yl)-phenylessigsäuremethylester (E/Z=3:1) als farbloses Öl.

Vorschrift 2

2-(3′-Phenyl-oxiran-2′-yl)-phenylessigsäuremethylester

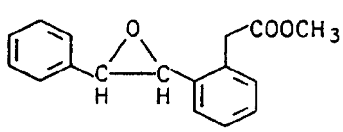

Zu einer Lösung von 6 g 2-(2′-Phenyl-ethen-1′-yl)-phenylessigsäuremethylester (E/Z=3:1) in 50 ml Methylenchlorid werden 7,8 g m-Chlorperbenzoesäure (ca. 50 %ig) gegeben. Nachdem das Reaktionsgemisch 12 Stunden unter Rückfluß erhitzt wurde, wird der entstandene Niederschlag abgesaugt und das Filtrat zweimal mit gesättigter Natriumhydrogencarbonat-Lösung und Wasser gewaschen. Die isolierte organische Phase wird über Natriumsulfat getrocknet, eingeengt und der verbleibende Rückstand aus Methyl-tert.-butylether umkristallisiert. Man erhält 3,1 g (48 %) Trans-2-(3′-Phenyl-oxiran-2′-yl)-phenylessigsäuremethylester mit dem Schmelzpunkt 118-119°C. Aus der Mutterlauge erhält man nach Chromatographie an Kieselgel (Essigester/n-Hexan=6:1) 2,8 g (44 %) 2-(3′-Phenyl-oxiran-2′-yl)-phenylessigsäuremethylester als 1:1-cis/trans-Gemisch.

Beispiel 1

2-[2-(3′-Phenyl-oxiran-2′-yl )-phenyl]-3-methoxy-acrylsäuremethyl-ester (Verbindung Nr. 1a)

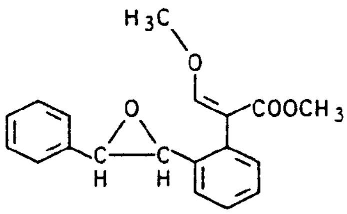

Zu einer Lösung von 10,2 g 2-[2-(2′-Phenyl-ethen-1′-yl)-phenyl]-3-methoxy-acrylsäuremethyl-ester in 80 ml Methylenchlorid werden 7,2 g m-Chlorperbenzoesäure (ca. 50 %ig) zugesetzt. Nachdem das Reaktionsgemisch zwei Tage unter Rückfluß erhitzt wurde, wird der entstandene Niederschlag abgesaugt und das Filtrat zweimal mit gesättigter Natriumhydrogencarbonat-Lösung und Wasser gewaschen. Die isolierte organische Phase wird über Natriumsulfat getrocknet, eingeengt und der verbleibende Rückstand aus Methyl-tert.-butylether umkristallisiert. Man erhält 6,2 g (58 %) Trans-2-[2-(3′-Phenyl-oxiran-2′yl)-phenyl]-3-methoxy-acrylsäure-methylester mit dem Schmelzpunkt 110-113°C.

Entsprechend Vorschrift 2 können die in der Tabelle 1 aufgeführten Zwischenprodukte hergestellt werden.

Entsprechend Beispiel 1 können die in der Tabelle 2 aufgeführten Verbindungen hergestellt werden.

Tabelle 1

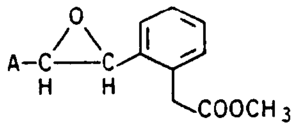

| Bsp. | A | Schmp./IR | Isomer* |
|---|---|---|---|
| 1 | $C_6H_5$ | 118-119°C | trans |
| 2 | $C_6H_5$ | | cis/trans=1:1 |
| 3 | 4-Cl-$C_6H_4$ | | |
| 4 | 3-Cl-$C_6H_4$ | | |
| 5 | 2-Cl-$C_6H_4$ | | |
| 6 | 2,4-$Cl_2$-$C_6H_3$ | | |
| 7 | 3,4-$Cl_2$-$C_6H_3$ | | |
| 8 | 2-Cl-4-F-$C_6H_3$ | | |
| 9 | 2-F-$C_6H_4$ | | |
| 10 | 4-F-$C_6H_4$ | | |
| 11 | 2,4-$F_2$-$C_6H_3$ | | |
| 12 | 2-Br-$C_6H_4$ | | |
| 13 | 4-Br-$C_6H_4$ | | |
| 14 | 4-$NO_2$-$C_6H_4$ | | |
| 15 | 4-$NH_2$-$C_6H_4$ | | |
| 16 | 2-$OCH_3$-$C_6H_4$ | | |
| 17 | 4-$OCH_3$-$C_6H_4$ | | |
| 18 | 4-$OC_6H_5$-$C_6H_4$ | | |
| 19 | 2-$CH_3$-$C_6H_4$ | | |
| 20 | 4-tert.-$C_4H_9$-$C_6H_4$ | | |
| 21 | 2-$CF_3$-$C_6H_4$ | | |
| 22 | 3-$CF_3$-$C_6H_4$ | | |
| 23 | 4-$CF_3$-$C_6H_4$ | | |
| 24 | 4-$C_6H_5$-$C_6H_4$ | | |
| 25 | 1-Naphthyl | | |
| 26 | 2-Naphthyl | | |
| 27 | H | | |
| 28 | $CH_3$ | | |
| 29 | $C_2H_5$ | | |
| 30 | $C_3H_7$ | | |
| 31 | iso-$C_3H_7$ | | |
| 32 | $C_6H_{12}$ | | |
| 33 | Cyclopropyl | | |
| 34 | Cyclopentyl | | |
| 35 | Cyclohexyl | | |

Tabelle 1 (Fortsetzung)

| Bsp. | A | Schmp./IR | Isomer* |
|---|---|---|---|
| 36 | 2-Cyclohexenyl | | |
| 37 | 3-Cyclohexenyl | | |
| 38 | 3-Pyridyl | | |
| 39 | 2-Furyl | | |
| 40 | 2-Thienyl | | |
| 41 | 3-Thienyl | | |
| 42 | 1,3-oxazol-5-yl | | |

* Die Zuordnung cis/trans bezieht sich auf die Konfiguration am Oxiranring

Tabelle 2

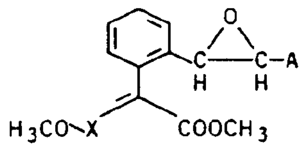

| Bsp. | A | X | Schmp./IR | Isomer* |
|---|---|---|---|---|
| 1a | $C_6H_5$ | CH | 110-113°C | trans |
| 2a | $C_6H_5$ | CH | | cis |
| 3a | $C_6H_5$ | N | | |
| 4a | 4-Cl-$C_6H_4$ | CH | | |
| 5a | 4-Cl-$C_6H_4$ | N | | |
| 6a | 3-Cl-$C_6H_4$ | CH | | |
| 7a | 2-Cl-$C_6H_4$ | N | | |
| 8a | 3-Cl-$C_6H_4$ | N | | |
| 9a | 2-Cl-$C_6H_4$ | CH | | |
| 10a | 2,4-$Cl_2$-$C_6H_3$ | CH | 90°C | trans |
| 11a | 2,4-$Cl_2$-$C_6H_3$ | N | | |
| 12a | 3,4-$Cl_2$-$C_6H_3$ | CH | | |
| 13a | 3,4-$Cl_2$-$C_6H_3$ | N | | |
| 14a | 2-F-$C_6H_4$ | CH | | |
| 15a | 2-F-$C_6H_4$ | N | | |
| 16a | 4-F-$C_6H_4$ | CH | | |
| 17a | 4-F-$C_6H_4$ | N | | |
| 18a | 2,4-$F_2$-$C_6H_3$ | CH | | |
| 19a | 2,4-$F_2$-$C_6H_3$ | N | | |
| 20a | 2-Br-$C_6H_4$ | CH | | |
| 21a | 2-Br-$C_6H_4$ | N | | |
| 22a | 4-Br-$C_6H_4$ | CH | | |
| 23a | 4-Br-$C_6H_4$ | N | | |
| 24a | 4-$NO_2$-$C_6H_4$ | CH | | |
| 25a | 4-$NO_2$-$C_6H_4$ | N | | |
| 26a | 4-$NH_2$-$C_6H_4$ | CH | | |
| 27a | 4-$NH_2$-$C_6H_4$ | N | | |
| 28a | 2-$OCH_3$-$C_6H_4$ | CH | | |
| 29a | 2-$OCH_3$-$C_6H_4$ | N | | |
| 30a | 4-$OCH_3$-$C_6H_4$ | CH | | |
| 31a | 4-$OCH_3$-$C_6H_4$ | N | | |
| 32a | 4-$C_6H_5O$-$C_6H_4$ | CH | | |
| 33a | 4-$C_6H_5O$-$C_6H_4$ | N | | |
| 34a | 2-$CH_3$-$C_6H_4$ | CH | | |
| 35a | 2-$CH_3$-$C_6H_4$ | N | | |

Tabelle 2 (Fortsetzung)

| Bsp. | A | X | Schmp./IR | Isomer* |
|---|---|---|---|---|
| 36a | 4-tert.-$C_4H_9$-$C_6H_4$ | CH | | |
| 37a | 4-tert.-$C_4H_9$-$C_6H_4$ | N | | |
| 38a | 2-$CF_3$-$C_6H_4$ | CH | | |
| 39a | 2-$CF_3$-$C_6H_4$ | N | | |
| 40a | 3-$CF_3$-$C_6H_4$ | CH | | |
| 41a | 3-$CF_3$-$C_6H_4$ | N | | |
| 42a | 4-$CF_3$-$C_6H_4$ | CH | | |
| 43a | 4-$CF_3$-$C_6H_4$ | N | | |
| 44a | 4-$C_6H_5$-$C_6H_4$ | CH | | |
| 45a | 4-$C_6H_5$-$C_6H_4$ | N | | |
| 46a | 3-$C_6H_5$-$C_6H_4$ | CH | 112°C | trans |
| 47a | 3-$C_6H_5$-$C_6H_4$ | N | | |
| 48a | 1-Naphthyl | CH | | |
| 49a | 1-Naphthyl | N | | |
| 50a | 2-Naphthyl | CH | | |
| 51a | 2-Naphthyl | N | | |
| 52a | H | CH | | |
| 53a | H | N | 65-67°C | |
| 54a | $CH_3$ | CH | | |
| 55a | $CH_3$ | N | | |
| 56a | $C_2H_5$ | CH | | |
| 57a | $C_2H_5$ | N | | |
| 58a | $C_3H_7$ | CH | | |
| 59a | $C_3H_7$ | N | | |
| 60a | iso-$C_3H_7$ | CH | | |
| 61a | iso-$C_3H_7$ | N | | |
| 62a | Cyclopropyl | CH | | |
| 63a | Cyclopropyl | N | | |
| 64a | Cyclopentyl | CH | | |
| 65a | Cyclopentyl | N | | |
| 66a | Cyclohexyl | CH | | |
| 67a | Cyclohexyl | N | | |
| 68a | 2-Cyclohexenyl | CH | | |
| 69a | 3-Cyclohexenyl | N | | |
| 70a | 2-Cyclohexenyl | N | | |
| 71a | 3-Cyclohexenyl | CH | | |
| 72a | 3-Pyridyl | CH | | |

Tabelle 2 (Fortsetzung)

| Bsp. | A | X | Schmp./IR | Isomer* |
|---|---|---|---|---|
| 73a | 3-Pyridyl | N | | |
| 74a | 2-Furyl | CH | | |
| 75a | 2-Furyl | N | | |
| 76a | 2-Thienyl | CH | | |
| 77a | 2-Thienyl | N | | |
| 78a | 3-Thienyl | CH | | |
| 79a | 3-Thienyl | N | | |
| 89a | 1,3-oxazol-5-yl | CH | | |
| 81a | 1,3-oxazol-5-yl | N | | |

* Die Zuordnung cis/trans bezieht sich auf die Konfiguration am Oxiranring

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine

(z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zübereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1a (Tab. 2) mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1a werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1a werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1a werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1a werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1a werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1a werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1a werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1a werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde N-Tridecyl-2,6-dimethylmorpholin (A) - bekannt aus DE 1 164 152 - benutzt.

Anwendungsbeispiel

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis zeigt, daß der Wirkstoff 1a (Tab. 2) bei der Anwendung als 0,05 %ige (Gew.%) Spritzbrühe eine bessere fungizide Wirkung zeigt (95 %) als der bekannte Vergleichswirkstoff A (65 %).

## Patentansprüche

1. Ester der allgemeinen Formel

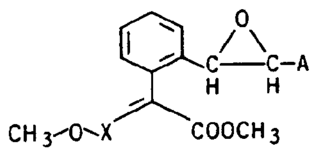

I,

in welcher

A Wasserstoff, $C_1$-$C_6$-Alkyl, $C_3$-$C_6$-Cycloalkyl, $C_5$-$C_6$-Cycloalkenyl, Naphthyl, Biphenyl, Pyridyl, Furyl, Thienyl, Oxazolyl, 1,3-Oxazolyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

X den Rest N oder CH bedeutet.

2. Phenylessigsäuremethylester der allgemeinen Formel II

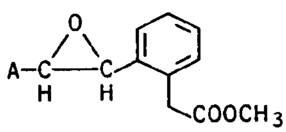

II,

in welcher A Wasserstoff, $C_1$-$C_6$-Alkyl, $C_3$-$C_6$-Cycloalkyl, $C_5$-$C_6$-Cycloalkenyl, Naphthyl, Biphenyl, Pyridyl, Furyl, Thienyl, Oxazolyl, 1,3- Oxazolyl oder Phenyl bedeutet, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können.

3. Ester der allgemeinen Formel I gemäß Anspruch 1, in denen A die Phenylgruppe bedeutet, welche unsubstituiert oder durch einen oder zwei Substituenten substituiert ist, welche gleich oder verschieden sind und Fluor, Chlor, Brom oder die Trifluormethylgruppe bedeuten.

4. Verfahren zur Herstellung der Ester der allgemeinen Formel I gemäß Anspruch 1, dadurch gekennzeichnet, daß man

a) eine Verbindung der Formel III

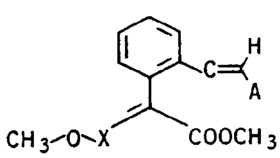

III,

in welcher A und X die oben angegebene Bedeutung haben, in das entsprechende Oxiran überführt,

oder

b) eine Verbindung der Formel II

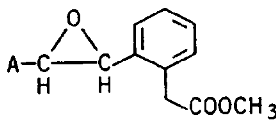

II,

in welcher A die oben angegebene Bedeutung hat, mit Ameisensäuremethylester in Gegenwart einer Base zur Reaktion bringt und das entstehende Enol mit einem Methylierungsreagenz umsetzt.

5. Verfahren zur Herstellung der Ester der Formel I gemäß Anspruch 1, in denen A den Rest H bedeutet, dadurch gekennzeichnet, daß man eine Verbindung der Formel IV

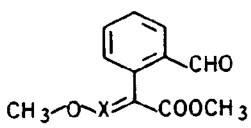

IV,

in welcher X den Rest CH bedeutet, mit einem Schwefelylid V

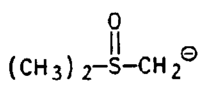

V,

umsetzt.

6. Fungizides Mittel, enthaltend einen Trägerstoff und eine fungizid wirksame Menge eines Esters der Formel I **gemäß Anspruch 1**.

7. Verfahren zur Bekämpfung von Pilzen, dadurch gekennzeichnet, daß man eine fungizid wirksame Menge eines Esters der Formel **gemäß Anspruch 1** auf die Pilze oder durch Pilzbefall bedrohte Materialien, Flächen, Pflanzen oder Saatgüter einwirken läßt.

8. Verbindung der Formel I gemäß Anspruch 1, dadurch gekennzeichnet, daß A Phenyl und X CH bedeutet.

9. Verbindung der Formel

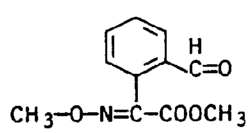

## Claims

1. An ester of the formula I

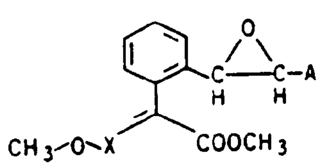

where A is hydrogen, $C_1$-$C_6$-alkyl, $C_3$-$C_6$-cycloalkyl, $C_5$-$C_6$-cycloalkenyl, naphthyl, biphenyl, pyridyl, furyl, thienyl, oxazolyl, 1,3-oxazolyl or phenyl, these radicals being unsubstituted or mono- to trisubstituted by halogen, nitro, phenoxy, amino, alkyl, alkoxy or haloalkyl of 1 to 4 carbon atoms in each case, and X is CH or N.

2. A methyl phenylacetate of the formula II

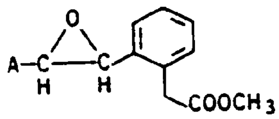

where A is hydrogen, $C_1$-$C_6$-alkyl, $C_3$-$C_6$-cycloalkyl, $C_5$-$C_6$-cycloalkenyl, naphthyl, biphenyl, pyridyl, furyl, thienyl, oxazolyl, 1,3-oxazolyl, or phenyl, these radicals being unsubstituted or mono- to trisubstituted by halogen, nitro, phenoxy, amino, alkyl, alkoxy or haloalkyl of 1 to 4 carbon atoms in each case.

3. An ester of the formula I as claimed in claim 1, where A is phenyl which is unsubstituted or has one or two identical or different substituents selected from the group comprising fluorine, chlorine, bromine and trifluoromethyl.

4. A process for preparing the esters of the formula I as claimed in claim 1, which comprises
a) converting a compound of the formula III

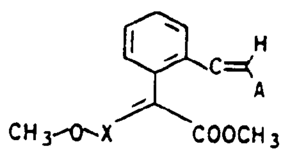

III,

where A and X have the abovementioned meanings, into the corresponding oxirane, or
b) reacting a compound of the formula II

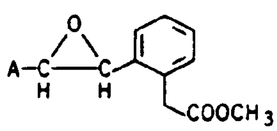

II,

where A has the abovementioned meanings, with methyl formate in the presence of a base, and reacting the resulting enol with a methylating reagent.

5. A process for preparing the esters of the formula I as claimed in claim 1, where A is H, which comprises reacting a compound of the formula IV

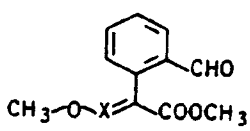

IV,

where X is CH, with a sulfur ylide V

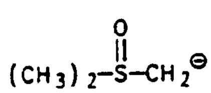

V,

6. A fungicidal composition containing a carrier and a fungicidally effective amount of an ester of the formula I as claimed in claim 1.

7. A method for controlling fungi, wherein a fungicidally effective amount of an ester of the formula I as claimed in claim 1, is allowed to act on the fungi, or on the materials, areas, plants or seed threatened by fungus attack.

8. A compound of the formula I as claimed in claim 1, where A is phenyl and X is CH.

9. A compound of the formula

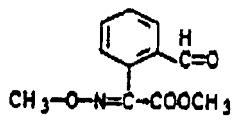

## Revendications

1. Esters de formule générale I

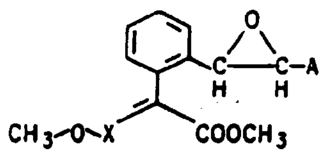

I,

dans laquelle

A représente un atome d'hydrogène, un alkyle en $C_1$-$C_6$, un cycloalkyle en $C_3$-$C_6$, un cycloalcényle en $C_5$-$C_6$, un naphthyle, un diphényle, un pyridyle, un furyle, un thiényle, un oxazolyle, un oxazolyle-1,3 ou un phényle ou ces restes peuvent être substitués de 1 à 3 fois par un halogène, un nitro, un phénoxy, un amino, un alkyle, un alcoxy ou un halogénoalkyle avec respectivement de 1 à 4 atomes de carbone,

X représente le reste N ou CH.

2. Esters méthyliques d'acide phénylacétique de formule générale II

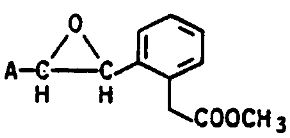

II,

dans laquelle

A représente un atome d'hydrogène, un alkyle en $C_1$-$C_6$, un cycloalkyle en $C_3$-$C_6$, un cycloalkényle en $C_5$-$C_6$, un naphtyle, un diphényle, un pyridyle, un furyle, un thiényle, un oxazolyle, un oxazolyle-1,3 ou un phényle, ou ces restes peuvent être substitués de 1 à 3 fois par un halogène, un nitro, un phénoxy, un amino, un alkyle, un alkoxy ou un halogénure d'alcyle, avec respectivement de 1 à 4 atomes de carbone.

3. Esters de formule générale I selon la revendication 1, dans lesquels A représente le groupe phényle, lequel n'est pas substitué ou est substitué par un ou deux substituts, lesquels sont identiques ou différents et représentent le fluor, le chlore, le brome, ou le groupe tri-fluorométhyle.

4. Procédé pour la fabrication des esters de formule générale I selon la revendication 1, caractérisé en ce que l'on transforme

a) un composé de formule III

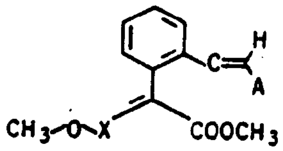

III,

dans laquelle A et X conservent la signification fixée plus haut, en l'oxirane correspondant, ou

b) en ce que l'on fait réagir un composé de formule II

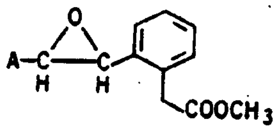

II,

dans laquelle A conserve la signification fixée plus haut, avec un ester méthylique d'acide formique en présence d'une base et que l'on fait réagir l'énol ainsi obtenu avec un réactif de méthylation.

**5.** Procédé pour la fabrication des esters de formule générale I selon la revendication 1, dans lesquels A représente le reste H, caractérisé en ce que l'on fait réagir un composé de formule IV

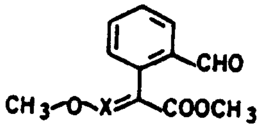

IV,

dans lequel X représente le reste CH, avec un thioylide V

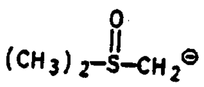

V.

**6.** Fongicide, contenant un composé porteur et une dose active de fongicide constitué d'un ester de formule I selon la revendication 1.

**7.** Procédé de lutte contre les champignons, caractérisé en ce que l'on fait agir une dose active de fongicide, constitué d'un ester de formule I selon la revendication 1, sur les champignons ou sur les matières, les surfaces, les plantes ou les semences menacées d'une attaque par les champignons.

**8.** Composé de formule I selon la revendication 1, caractérisé en ce que A représente un phényle et X un CH.

**9.** Composé de formule